# EUROPEAN PATENT APPLICATION

(11) **EP 1 428 879 A1**
(43) Date of publication of application: **16.06.2004**
(21) Application number: 02730901.2
(22) Date of filing: 04.06.2002
(51) Int. Cl.: C12N 15/12, C12N 15/11, A61K 38/02, A61K 39/00, A61K 48/00

(54) **POLYNUCLEOTIDE VACCINE**

(30) Priority: 25.06.2001 JP 2001191334
(71) Applicant: Anges MG Inc., Toyonaka-shi, Osaka 560-0082 (JP)
(72) Inventor: SHIKU, Hiroshi, Tsu-shi, Mie 514-0061 (JP)
(74) Representative: Chadwick, Mark Craig
(86) International application number: PCT/JP2002/005486
(87) International publication number: WO 2003/000894

(57) **Abstract**

The present invention relates to a DNA vaccine for developing tumor-specific immunity. Specifically, the invention relates to a DNA vaccine comprising antigens recognized by CD4⁺ helper T cells and.CD8⁺ cytotoxic T cells, respectively. Preferably, the antigen recognized by CD4⁺ helper T cells is a molecule identified by the SEREX method and that recognized by CD8⁺ cytotoxic T cells is a tumor antigen. Furthermore, so that the antigens recognized by CD4⁺ helper T cell and CD8⁺ CTL are expressed and presented on the same cell, the expression vectors preferably are immobilized on the same gold particle and administered into the cell by gene gun; however, this invention is not restricted thereto. The vaccine can also be used as a pharmaceutical agent.

## Description

### Technical Field

The present invention relates to a vaccine for developing tumor-specific immunity *in vivo.* Specifically, the invention relates to a polynucleotide vaccine comprising expression vectors encoding an antigen recognized by CD4⁺ helper T cells and an antigen recognized by CD8⁺ cytotoxic T cells (CTL). The antigen recognized by the CD4⁺ helper T cells is preferably a molecule detected via serological identification of antigen by the recombinant cDNA expression cloning (SEREX) method. On the other hand, the antigen recognized by CD8⁺ CTL is preferably a tumor antigen. Furthermore, so that the antigens recognized by CD4⁺ helper T cells and CD8⁺ CTL are expressed and presented on the same cell, the expression vectors are preferably immobilized on the same gold particle and administered into the cell by a gene gun; however, the invention is not restricted thereto.

### Background Art

For the past several years, cancer has been the leading cause of death in Japan. In addition to surgery, radiotherapy and chemotherapy, immunotherapy is the fourth option for cancer therapy. An *in vivo* system for immune response is utilized in immunotherapy. The immune response is elicited and controlled by the interaction amongst B lymphocytes, T lymphocytes, antibodies and antigen presenting cells (APC). First, an exogenous antigen is processed in the APC, and then it is presented in a form bound to the major histocompatibility complex (MHC) class 1 or class 2 to helper T cells. Upon the recognition by helper T cells of this exogenous antigen, T cells are activated and cytokines are secreted. The secreted cytokines help differentiation of antigen-stimulated B cells into antibody-forming cells and at the same time promote the differentiation of killer T cells. Finally, cells presenting antigens are eliminated by the secreted antibodies and by the activated killer T cells. This is how cellular and humoral responses operate in order to eliminate exogenous antigens.

The elimination process of antigen expressing cells by T cells can be broadly classified in three groups: 1) humoral immunity (activated helper T cells stimulate proliferation/differentiation of specific B cell clones, antibodies are produced that recognize and eliminate antigens); 2) specific cellular immunity (activated helper T cells induce cytotoxic T cells (CTL) that react with specific antigens and the CTL directly react to the target); and 3) non-specific cellular immunity (activated helper T cells induce non-specific natural killer cells, activated macrophages, etc. and these cells function to eliminate antigens). As described above, T cells play a central role in recognizing target antigens to elicit an immune response.

Regarding tumor rejection, antitumor immune responses in host cells have been known to be induced by appropriate immunization using syngeneic or self-derived tumor cells or fractions thereof (L. Gross, Cancer Res. 3: 326-333 (1943); E.J. Foley, Cancer Res. 13: 835-837 (1953) ; R.T. Prehn and J.M. Maln, J. Natl. Cancer Inst. 18: 769-778 (1957); G. Klein et al., Cancer Res. 20: 1561-1572 (1980); L. Old et al., Ann. N.Y. Acad. Sci. 101: 80-106 (1962); A. Globerson and M. Feldman, J. Natl. Cancer Inst. 32: 1229-1243 (1964)). The role of CD8⁺ and CD4⁺ T cells in these tumor systems has been of enormous interest (R.J. North, Adv. Immunol. 35: 89-155 (1984) ; P.D. Greenberg, Adv. Immunol. 49: 281-355 (1991); D.M. Pardoll and S.L. Topalian, Curr. Opin. Immunol. 10: 588-594 (1998)). CD8⁺ T cells from specifically immunized mice are reported to be capable of destroying tumor target cells *in vitro* (H. Wagner et al., Adv. Cancer Res. 31: 77-124 (1980)). Furthermore, it has been reported that adoptive transfer of CD8⁺ T cells from immunized donors confers resistance to tumor transplants to naive mice (R.J. North, Adv. Immunol. 35: 89-155 (1984) ; P.D. Greenberg, Adv. Immunol. 49: 281-355 (1991) ; C. J.M. Melief, Adv. Cancer Res. 58: 143-175 (1992)). In addition, anti-CD8⁺ antibodies are known to abolish resistance to tumor transplantation in preimmunized mice (E. Nakayama and A. Uenaka, J. Exp. Med. 161: 345-355 (1985); X.G. Gu et al., Cancer Res., 58: 3385-3390 (1998) ; Y. Noguchi et al. , Proc. Natl. Acad. Sci. USA 92: 2219-2223 (1994)). Over the past decade, MHC class I binding peptides derived from tumor cells of mice and human that are recognized by CD8⁺ T cells have been reported (T. Boon et al. , Annu. Rev. Immunol. 12: 337-368 (1994); S.A. Rosenberg, Immunity 10: 281-287 (1999)).

Two forms exist for tumor antigens (target molecules on tumor cells). These are: (1) tumor peptides presented by MHC class I molecules, the target molecules of CD8⁺ CTL that is the leading character of cellular immunity; and (2) the target molecule of humoral immunity (antibody) that is expressed on the cell membrane of tumors is called tumor-associated antigen. Since a human tumor antigen recognized by a T cell has been defined at the genetic level, various human tumor rejection antigens have been discovered. Vaccination therapy is a specific immunotherapy that uses a tumor rejection antigen and evident antitumor effect has been confirmed for the therapy. Furthermore, potentiation of immunotherapeutic effect by the combined use of cytokines and dendritic cells pulsed with antigenic peptides, or introduced with antigen genes, has been attempted. Moreover, recently, DNA vaccines have been tested in the art.

A number of approaches to augment the helper action of CD4⁺ T cells have been attempted (D. Pardoll and S.L. Topalian, Curr. Opin. Immunol. 10: 588-594 (1998); R.F. Wang, Trends Immunol. 5: 269-276 (2001)). Earlier methods fall into one of three categories. One method involves modification of immunizing antigens itself. For example, haptenizing the antigen (Y. Mizushima et al., J. Natl. Cancer Inst. 74: 1269-1273 (1985)), linking heterologous immunogenic peptides directly onto the antigen (R.W. Chesnut et al., Vaccine Design, eds. M.F. Powell and M.J. Newman (Plenum, New York) 847-874 (1995) ; J. Rice et al., J. Immunol. 167: 1558-1565 (2001)), etc. The second method is co-immunization with tumor antigens and molecules with strong helper determinants (R. Romieu et al., J. Immunol. 161: 5133-5137 (1998) ; N. Casares et al. , Eur. J. Immunol. 31: 1780-1789 (2001)), such as viral vectors encoding tumor antigens (M. Wang et al., J. Immunol. 154: 4685-4692 (1995)). The third method utilizes molecular signals such as CD40 ligand (J.P. Ridge et al., Nature (London) 393: 474-478 (1998) ; S.R.M. Bennett et al. , Nature (London) 393: 478-480 (1998); S.P..Schoenberg et al., Nature (London) 393: 480-483 (1998)) and other stimulatory/co-stimulatory signals (A. Porgador et al., J. Exp. Med. 188: 1075-1082 (1998)) involved in the helper function of CD4⁺ T cells and in modulating the interaction of APCs with CD4⁺ T cells. The discovery of such signals appears to provide methods to augment the response of CD8⁺ T cells.

Antibodies have been generally relegated to a minor role in antitumor effector functions. However, tumor antigens like NY-ESO-1 elicit a strong integrated immune response involving both cellular and humoral immunities (Y.-T. Chen et al. , Proc. Natl. Acad. Sci. USA 94: 1914-1918 (1997); E. Jager et al., J. Exp. Med. 187: 625-630 (2000); E. Jager et al., Proc. Natl. Acad. Sci. USA 97: 12198-12203 (2000)). Recently, M. Pfreundschuh and his colleagues developed a method called SEREX (Y.-T. Chen et al., Proc. Natl. Acad. Sci. USA 94: 1914-1918 (1997)) to identify tumor-associated antigens that may serve as vaccines against tumors. This method involves screening of cDNA expression libraries of human tumors with human sera. More than 1800 kinds of genes identified by SEREX are registered in the SEREX database on the. internet (www.licr.org/SEREX.html).

However, there are still unsolved problems. Some of the problems include the kind of adjuvant or APC to be used for effectively inducing tumor-specific immunity with the identified antigenic peptides/DNAs to finally achieve complete recovery from tumor; or dealing with the escape of tumors from the immune system.

Helper T cells are often reported to be necessary for quantitative/qualitative amplification of CTL. However, the characteristics of antigen molecules recognized by these T cells and their functional impact on antitumor immune responses are still largely unknown (P.D. Greenberg, Adv. Immunol. 49: 281-355 (1991); D.M. Pardoll and S.L. Topalian, Curr. Opin. Immunol. 10: 588-594 (1998); S.R. Bennett et al., J. Exp. Med. 186: 65-70 (1997); R.F. Wang, Trends Immunol. 5: 269-276 (2001) ; C. Fayolle et al. , J. Immunol. 147: 4069-4073 (1991); M. Shiral et al. , J. Immunol. 152: 1549-1556 (1994); K. Hung et al., J. Exp. Med. 188: 2357-2368 (1998); F. Ossendorp et al., J. Exp. Med. 187: 693-702 (1998); Y. Shen and S. Fujimoto, Cancer Res. 56: 5005-5011 (1996); T. Nishimura et al. , J. Exp. Med. 190: 617-627 (1999) ; D.R. Surman et al. , J. Immunol. 164: 562-565 (2000); A. Franco et al., Nat. Immunol. 1: 145-150 (2000); C.N. Baxevanis et al., J. Immunol. 164: 3902-3912 (2000); F. Fallarino et al., J. Immunol. 165: 5495-5501 (2000); A.L. Marzo et al., Cancer Res. 59: 1071-3390 (1999) ; A.L. Marzo et al., J. Immunol. 165: 6047-6055 (2000)). The current hypothesis for serial intercellular interaction amongst helper T cells, CTLs and APCs points to the possibility that helper T cells related to antitumor immune response can recognize diverse antigens of a wide range (J.P. Ridge et al., Nature 393: 474-478 (1998); S.R.M. Bennett et al., Nature 393: 478-480 (1998); S.P. Schoenberger et al., Nature 393: 480-483 (1998); Z. Lu et al., J. Exp. Med. 191: 541-550 (2000)).

Great progress is being made in the analysis of humoral immune response in human and murine tumors by the above-described SEREX method (Y.-T. Chen et al., Proc. Natl. Acad. Sci. USA 94: 1914-1918 (1997) ; E. Jager et al., J. Exp. Med. 187: 625-630 (2000) ; E. Jager et al. , Proc. Natl. Acad. Sci. USA 97: 12198-12203 (2000); U. Sahin et al., Proc. Natl. Acad. Sci. USA 92: 11810-11813 (1995); L.J. Old and Y.-T. Chen, J. Exp. Med. 187: 1163-1167 (1998); Y.-T. Chen, "Principle and Practice of the Biologic Therapy of Cancer", ed. S.A. Rosenberg (Lippincott Williams & Wilkins, Philadelphia) 557-570 (2000) ; T. Ono et al. , Int. J. Cancer 88: 845-851 (2000)). Complete sequence determination of SEREX-defined genes show that many of them share the same sequence with the wild-type sequence, i.e., there are no amino acid substitutions included (L.J. Old and Y.-T. Chen, J. Exp. Med. 187: 1163-1167 (1998); Y.-T. Chen, "Principle and Practice of the Biologic Therapy of Cancer", ed. S.A. Rosenberg (Lippincott Williams & Wilkins, Philadelphia) 557-570 (2000)). Therefore, these molecules do not exhibit immunogenicity due to mutations. Furthermore, although some SEREX antigens show restricted tumor expression in normal tissues (e.g., cancer/testis antigens, melanocyte differentiation antigens, etc.), most of the SEREX-defined antigens are ubiquitously expressed. However, high-titered antibodies to these wild-type molecules are present more in the serum samples of associated and non-associated cancer patients as compared to normal healthy subjects. The current hypothesis is that amplified expression of these tumor products serves as the immunogenic stimulus for eliciting humoral immunity. Since all of these molecules are detected by antibodies of the IgG class, these wild-type molecules imply recognition by CD4⁺ helper T cells. With regard to the above information, the present inventor examined in the present invention whether tumor-specific CD8⁺ CTL can be amplified by activating CD4⁺ helper T cells via immunogenic wild-type molecules of tumor cells. Namely, examined the involvement of the molecules in antitumor immune response.

DNA vaccines have been shown to induce both humoral and cellular immune responses upon intramuscular administration of naked DNA. The precise mechanism of induction of immune response by DNA vaccines is obscure (see, Pardoll et al., Immunity 3: 165-169 (1995)). However, its effectiveness is indicated by the induction of humoral and cellular immunities. This result indicates the expression of naked DNA following administration of a DNA vaccine, and that peptide products of the naked DNA are presented as antigens with both the MHC class I and class II proteins.

A T cell receptor on CTL recognizes an exogenous peptide derived from virus, bacteria, etc., bound to MHC class I and/or class II molecules as an antigen. Then, reactions such as the production of various lymophokines and cell proliferation are known to be promoted to finally kill cells infected with the virus, bacteria, etc. Irrespective of their location in the original pathogen, these antigenic peptides are processed fragments that were intracellularly imported into APC or other cells. Known methods for artificial generation of CTL response include those using replication vectors that produce protein antigens in cells (J.R. Bennink and J.W. Yewdell, Curr. Top. Microbiol. Immunol. 163: 153 (1990); C.K. Stover et al., Nature 351: 456 (1991) ; A. Aldovini and R.A. Young, Nature 351: 479 (1991); R. Schfer et al., J. Immunol. 149: 53 (1992); C.S. Hahn et al., Proc. Natl. Acad. Sci. USA 89: 2679 (1992) ) and methods wherein peptides are introduced into the cytosol (F.R. Carbone andM.J. Bevan, J. Exp. Med. 169: 603 (1989) ; K. Deres et al. , Nature 342: 561 (1989) ; H. Takahashi et al., Nature 344: 873 (1990); D.S. Collins et al., J. Immunol. 148: 3336 (1992) ; M.J. Newman et al., J. Immunol. 148: 2357 (1992)).

Furthermore, a method for inoculating a vertebrate with naked polynucleotide as a vaccine has been discussed (WO90/11092 (October 4, 1990)). Calcium chloride-precipitated DNA is known to be expressed via intravenous or intramuscular administration (N. Benvenisty and L. Reshef, Proc. Natl. Acad. Sci. USA 83: 9551-9555 (1986)). Moreover, in mice it was shown that DNA expression vector is incorporated into myocytes and expressed in the cell upon intramuscular injection of the vector (J.A. Wolff et al., Science 247: 1465 (1990); G. Ascadi et al., Nature 352: 815 (1991)). According to this method, the vector was sustained as an episome and did not replicate. However, permanent expression of the vector was observed following injection into the skeletal muscle of rat, fish and primate, as well as cardiac muscle of rat (H. Lin et al., Circulation 82: 2217 (1990); R.N. Kitsis et al., Proc. Natl. Acad. Sci. USA 88: 4138 (1991) ; E. Hansen et al., FEBS Lett. 290: 73 (1991) ; S. Jiao et al., Hum. Gene Therapy 3: 21 (1992); J.A. Wolff et al., Human Mol. Genet. 1: 363 (1992)). It was further reported that presentation of epitopes by B7 and MHC on the surface of APC play similar roles in the activation of CTL during tumor elimination (Edington, Biotechnology 11: 1117-1119 (1993)). When a MHC molecule on the surface of APC presents an epitope to a T cell receptor, a B7 expressed on the surface of the same APC binds to CTLA-4 or CD28 and functions as the second signal. As a result, CD4⁺ helper T cells that emit signals for increasing APC destroying CD8⁺ T cells rapidly proliferate.

For immunization with DNAs, the DNAs do not necessary have to be administered intramuscularly. For example, Tang et al. demonstrate that anti-bovine growth hormone (BGH) antibodies are produced in mice following administration of BGH-coated gold particles into the skin (Tang et al., Nature 356: 152-154 (1992)). Apart from skin, it is reported that muscular, adipose and mammary gland tissues of live animals can be transfected with DNAs (Furth et al., Analytical Biochemistry 205: 365-368 (1992)). Various methods for introducing nucleic acids are also reviewed (T. Friedman, Science 244: 1275-1281 (1989)). WO93/17706 describes a method of vaccine inoculation of an animal against a virus that comprises the steps of coating a carrier particle with a gene construct, and then administering the coated particle into a cell of the animal. Furthermore, DNA immunization against herpes virus (Cox et al., J. Virol. 67: 5664-5667 (1993)) has been reported. In addition, DNA vaccines and methods for producing and administering them are also described in US Patent No. 4,945,050, US Patent No. 5,589,466, and WO94/16737.

### Disclosure of the Invention

An objective of the present invention is to provide effective immunotherapy against tumors. Additional objectives include providing a radical treatment for early stage cancers, therapy for suppressing postoperative recurrence or metastasis of cancers, and treatment for patients detected to have inoperable tumors, and for whom radical and chemical treatments were ineffective.

The present invention relates to a vaccine for eliciting tumor-specific immunity. The invention is based on the finding that tumor-specific immunity can be induced using an expression vector encoding an antigen recognized by CD4⁺ T cells and an expression vector encoding an antigen recognized by CD8⁺ T cells as a polynucleotide vaccine. Thus, the present invention relates to compositions comprising expression vectors encoding antigens recognized by CD4⁺ T cells and CD8⁺ T cells, respectively. Specifically, the antigen recognized by CD4⁺ helper T cells is a molecule determined by the SEREX method, and that recognized by CD8⁺ CTL is a tumor antigen. Furthermore, the antigens recognized by CD4⁺ helper T cells and CD8⁺ CTL are immobilized on the same carrier particle and administered into a cell to express and present them onto the same cell; however, the invention is not restricted thereto.

As described herein, the phrase "antigen recognized by CD4⁺ helper T cells" refers to a polypeptide that comprises a portion that serves as an epitope of a glycoprotein expressed on mature helper T cell or precursor cell thereof. When a T cell receptor recognizes an antigen presented by MHC class II, CD4 binds to the MHC class II β2 domain and thus enhances the ability of the T cell to recognize antigens. CD4 further transmits signal into the cell, and functions as a molecule to promote proliferation and secretion of cytokines. According to the present invention, molecules identified by SEREX are particularly preferred among the molecules recognized by CD4⁺ helper T cells. Such molecules include a heat shock protein Dna J-like 2, DNA ligase 1, galectin 1, poly(A) binding protein, *Homo sapiens* hexamethylene-bis-acetamide-inducible (XM_008348), human retinoic acid-responsive protein (U50383), *H. sapiens* hepatitis delta antigen interacting protein A (DIPA) (XM_006503), *H. sapiens* cDNA FLJ20644 fis clone KAT002588 (FLJ20644fis) (AK000651), etc.; but are not restricted thereto. The phrase "SEREX-defined molecule" herein refers to such molecules identified by SEREX. In addition, the phrase "immunogenic wild-type cellular molecule" herein refers specifically to the molecules identified by SEREX that are recognized by CD4⁺ helper T cells as described above.

Furthermore, herein, the phrase "antigen recognized by CD8⁺ cytotoxic T cells (CTL)" refers to polypeptides comprising a portion that serves as an epitope of transmembrane glycoprotein CD8 expressed on CTL and precursor cells thereof. When a T cell receptor recognizes an antigen presented by MHC class I, it binds to the α3 domain of MHC class I. The antigen recognizing ability of the T cell is enhanced by the binding and the signal is transmitted into the cell. The "antigen recognized by CD8⁺ cytotoxic T cells" is a molecule having the function to enhance the proliferation and cytotoxic activity of the T cell via such pathways. Particularly preferred molecules are tumor antigens, including antigens related to tumors and antigens specifically expressed on particular tumor cells. These molecules include polypeptides such as mutant MAP kinase ERK2 (mERK2), HER2 and Dna J-like 2. HER2 is a tumor rejection antigen of congenital sarcoma CMS17HER2, a cell line derived from CMS5a generated by transfection with human HER2 cDNA (c-erb-2/HER2/neu cDNA) (Y. Nagata et al., J. Immunol. 159: 1336-1343 (1997) ; X. Gu et al. , Cancer Res. 58: 3385-3390 (1998) ; Y. Ikuta et al. , Int. J. Cancer 87: 553-558 (2000) ; T. Okugawa et al. , Eur. J. Immunol. 30: 3338-3346 (2000)). The HER2 is preferred as the antigen recognized by CD8⁺ cytotoxic T cells (CTL). The full-length sequence or immunogenic fragments of the HER2 may be used in the present invention. A 9mer peptide, HER2 p63-71(T) peptide (amino acid sequence "TYLPTNASL"), has been identified as a tumor rejection antigen against CMS17HER2 or as the target of CD8⁺ K^{d}-restricted cytotoxic T cells (Y. Nagata et al., J. Immunol. 159: 1336-1343 (1997) ; X. Gu et al. , Cancer Res. 58: 3385-3390 (1998); Y. Ikuta et al., Int. J. Cancer 87: 553-558 (2000); T. Okugawa et al., Eur. J. Immunol. 30: 3338-3346 (2000)). Fragments comprising such antigenic sites are particularly preferred. 'Furthermore, the combination of HER2 and Dna J-like 2 is particularly preferred as the antigens recognized by CD8⁺ cytotoxic T cells (CTL) of the present invention.

Polynucleotides encoding the antigens of the present invention are not restricted and may be DNA, RNA, etc., as long as they can elicit the desired immune response by administering them to the host animal according to the method of the present invention. The polynucleotides encoding antigens of the present invention may be those whose nucleotide sequence is artificially modified by one or more amino acids deletions, substitutions, insertions and/or additions according to known methods such as site-directed mutagenesis (see, edited by Ausubel et al., Current Protocols in Molecular Biology, John Wiley & Sons, Section 8.1-8.5 (1987)) as long as the polypeptide encoded by the polynucleotide can generate desired immune response in the host. Furthermore, as long as it can elicit a desired immune response in the host, the polynucleotide may encode polypeptides having mutations that exist in nature. Such mutants existing in nature may be isolated utilizing known hybridization techniques (see, edit. Ausubel et al. , Current Protocols in Molecular Biology, John Wiley & Sons, Section 6.3-6.4 (1987)) and gene amplification techniques (PCR) (see, edit. Ausubel et al., Current Protocols in Molecular Biology, John Wiley & Sons, Section 6.1-6.4 (1987)).

Moreover, if a gene encoding an antigenic protein is known, one skilled in the art can readily analyze hydrophobic and/or hydrophilic regions within the amino acid sequence of the protein (Kyte and Doolittle, J. Mol. Biol. 157: 105-122 (1982)), analyze its secondary structure (Chou and Fasman, Ann. Rev. Biochem. 47: 251-276 (1978)), and synthesize peptides with the predicted amino acid sequence to determine its antigenicity by PEPSCAN (Nature 314 (1985) ; Published Japanese Translation of International Publication No. Sho 60-500684) , etc. Thus, a polynucleotide encoding a peptide fragment that comprises the epitope site determined base on the above-described method may be prepared by chemical synthesis and so on, to be used as the antigen of the present invention in an expression vector.

The expression vector used in the present invention is a recombinant vector wherein the antigen gene of the present invention is inserted therein. The vectors to insert the antigen gene include plasmids, phages, cosmids, viruses, and other conventional vectors in the technical field of the present invention. Those skilled in the art can construct various plasmids and vectors based on well known techniques (edited by Sambrook et al. Molecular Cloning: A Laboratory Manual (Cold Spring Harbor Laboratory, N.Y. (1989) and edited by Ausubel et al., Current Protocols in Molecular Biology, John Wiley & Sons, Section 6.1-6.4 (1987)).

Herein, factors such as promoter and terminator regulating the expression in the host can be appropriately selected by those skilled in the art from known regulatory sequences depending upon the specific type of host and object, and desired arrangement (i.e., upstream and/or downstream to the antigen gene). Therefore, regulatory sequences derived from the antigen or heterogeneous regulatory sequences may be used in the present invention. Furthermore, if needed, markers such as an antibiotic resistance marker may be used as the expression vector in the present invention. Commercially available multiple vectors may be used. However, it is preferred to delete nonessential polynucleotide sequences from the vector in the present invention. In addition, the polynucleotide encoding an antigen recognized by CD4⁺ helper T cells and that encoding an antigen recognized by CD8⁺ CTL used in the present invention may be included in different expression vectors or constructed to be expressed from a single vector as long as they can be controlled for their expression on the same cell.

Upon introduction into the tissue of an animal, the vaccine of the present invention induces in vivo expression of the present antigen and elicits the desired immune response. Various methods are known for introducing nucleic acids in vivo (T. Friedman, Science 244: 1275-1281 (1989)). Any method may be used as long as the antigens of the present inventions are expressed in vivo and elicit the desired immune response.

The compositions of the present invention are useful as vaccines and can be used as naked plasmids. They may be packaged into a liposome, formed as various virus vectors including retrovirus vectors, adenovirus vectors, vaccinia virus vectors, poxvirus vectors, adeno-associated virus vectors and HVJ (hemmagglutinating virus of Japan) vectors (see, e.g. , K. Adolph "Virus genomic methods", CRC Press, Florida (1996)), or coated on beads (carriers) such as colloidal gold particles. Preferably, the vectors expressing the antigens recognized by CD4⁺ helper T cells and CD8⁺ CTL, respectively, are in the form adhered to a carrier particle, such as'gold particle, for introduction into the body by gene gun, etc.; however, the present invention is not limited thereto. Methods for coating polynucleotides on a carrier particle are known in the art (see, for example, W093/17706). Finally, the polynucleotides are prepared in a solution such as physiological saline adapted for *in vivo* administration. The composition of the present invention may be used as a vaccine, in combination with adjuvants such as proteins or other carriers that are known in the art for enhancing immune reactions. Moreover, agents such as calcium ion that help the intracellular uptake of plasmids may be used in combination. In addition, pharmaceutically accepted agents that achieve easier transfection may be combined as required.

The polynucleotide vaccine of the present invention may be administered by any method as long as it generates an immune response within the host animal. Preferably, the composition of the present invention is administered at a dose sufficient to induce immune response in the host animal by appropriate methods including injections, infusion or gas-induced particle bombardment method (using gene gun, etc.) via parenteral routes, such as intravenous, intraperitoneal, subcutaneous, intradermal, via adipose tissue, via mammary gland tissue, inhalation or intramuscular, or mucosal routes in the form of nasal drops. Furthermore, a host animal may be immunized by administering the present composition into a blood cell, bone marrow-derived cell (e.g., APC) etc. by *ex vivo* liposome transfection, particle bombardment method, virus infection, etc., and then reintroducing the cell into the animal. Among the above-mentioned administration methods, the gene transformation method using accelerated particles is described in US Patent No. 4,945,050 and devices base on modified methods thereof are commercially available (BioRad Laboratories).

The specific type of host animal of the present invention is not limited as long as the tumor immune response of the animal is enhanced by the composition of the present invention. Specific examples include mammals, such as mice, rats, bovines, pigs, primates like monkey and human, etc. Preferable host animals of the present invention include primates, particularly human.

The dose of the composition of the present invention depends on the immunogenicity in the host of the ingredient comprises in the composition. However, those skilled in the art can determine appropriate dose required for immunization by administering a determined dose of the composition into a test animal and measuring the antibody titer by assay methods such as ELISA, detecting CTL response by measurement of chromium release, etc., or observing immune response by detecting Th response using cytokine release measurement. Those skilled in the art will recognize that the immunogenicity of the ingredient in the composition depends also on the intensity of the regulatory sequence, such as transcription and translation promoters, used in the expression vector of the present invention. Furthermore, those skilled in the art can readily adjust the dose of the composition of the present invention based on the specific expression vector used.

### Brief Description of the Drawings

Fig. 1 depicts graphs showing the increase in the number of 9m peptide specific CD8⁺ T cells that are generated by the immunization using mERK2 and SEREX-defined molecules. Fig. 1b shows the results when human SEREX-defined molecules were used.
Fig. 2 depicts graphs showing that the enhancement of 9m peptide-specific CD8⁺ T cells induced by SEREX-defined molecules requires co-presentation of 9m peptide (panel a) and existence of CD4⁺ T cells (panel b).
Fig. 3 depicts graphs showing that the helper activity due to CD4⁺ T cells recognizing SEREX-defined antigen molecules enhances 9m-specific CD8⁺ T cells in both the primary and secondary responses.
Fig. 4 depicts graphs showing that SEREX-defined molecules recognizing CD⁺4 T cells co-presented with HER2 p63 peptide induce HER2 p63-specific CD8⁺ T cells.
Fig. 5 depicts graphs showing *in vivo* preventive and therapeutic effects against pulmonary metastasis of immunization using mERK2 or 147HER2 and Dna J-like 2.

### Best Mode for Carrying out the Invention

Hereinafter, the present invention is described in detail below with reference to Examples. However, it should not be construed as being limited thereto.

### [Example 1] Identification of tumor-derived immunogenic molecules

To identify immunogenic molecules in murine tumor cells, cDNA λ phage. libraries prepared from BALB/c origin 3-methylchoranthrene-induced sarcoma CMS5, CMS2, CMS8 and CMS13 lines (A.B. DeLeo et al. , J. Exp. Med. 146: 720-734 (1977)), andCMS5a and CMS5m subcloned from CMS5 were screened by SEREX (U. Sahin et al., Proc. Natl. Acad. Sci. USA 92: 11810-11813 (1995)) using sera samples (IgG antibody) from syngeneic mice bearing cognate tumor lines. ' Among the array of genes detected, four of the most frequently detected gene products were Mus heat shock protein Dna J-like 2 (AF055664), Mus galectin-8 (hereinafter referred to as galecting-8) (AF218069), Mus DNA ligase 1 (U19604) and Mus poly(A) binding protein cytoplasmic 1 (hereinafter referred to as poly(A))(X65553). Analyses of the coding sequences of these genes did not reveal any mutations as compared to registered sequences in GenBank (Table 1). These highly immunogenic tumor antigens were selected for further study. In addition, three cDNAs that were consistently negative in repetitive SEREX screenings, Mus sorting nexin 1(AB019214), Mus glucose-regulated protein (D78645) and Mus Cctz-1 gene for chaperon containing TCP-1-zeta-1 subunit (AB022159) were included to represent nonimmunogenic molecules derived from tumor cells.

Furthermore, immunogenic molecules were detected with serum of idiopathic thrombocytopenic purpura patients using cDNAs encoding antigens of UT-7/TPO (human megakaryoblastic leukemia cell line) (N. Komatsu et al., Blood 87: 4552-4560 (1996)). For further study, (1) *Homo sapiens* hexamethylene-bis-acetamide-inducible (XM_008348), (2) human retinoic acid-responsive protein (U50383), (3) *H. sapiens* hepatitis delta antigen interacting protein A (DIPA) (XM_006503) and (4) *H. sapiens* cDNA FLJ20644 fis clone KATO2588 (FLJ20644fis) (AK000651) were selected as SELEX-defined human genes.

**Table 1.**

| Antigens(accession number) | Size (bp) | Source (cDNA expression libraries) |
|---|---|---|
| Murine molecules | | |
| Immunogenic* | | |
| heat shock protein, Dna J-like 2 (AF55664) | 2,242 | CMS5a and CMS2 |
| DNA ligase 1 (U19604) | 3,172 | CMS13 |
| galectin-8 | 1,086 | CMS2 and CMS7 |
| poly(A) binding protein, cytoplasmic 1 (X65553) | 2,244 | CMS8 |

| Nonimmunogenic** | | |
|---|---|---|
| sorting nexin 1 (AB019214) | 2,007 | CMS5a |
| glucose-regulated protein (D78645) | 2,408 | CMS5a |
| Cctz-1 gene for chaperon containing | 19,505 | CMS5a |
| TCP-1-zeta-1 subunit (AB022159) | | |

| Human molecules | | |
|---|---|---|
| Immunogenic*** | | |
| *Homo sapiens* HMBA-inducible (XM_008348) | 3,594 | UT-7/TPO |
| human retinoic acid-responsive protein (U50383) | 2,520 | UT-7/TPO |
| *H. sapiens* hepatitis delta antigen protein A (XM_006503) | 997 | UT-7/TPO |
| *H. sapiens* cDNA FLJ20644 fis, clone KAT002588 (AK000651) | 1,781 | UT-7/TPO |
| HMBA; hexamethylene-bis-acetamide | | |

| | | |
|---|---|---|
| *Detected by syngeneic antibody from tumor-bearing mice in SEREX analysis of cDNA expression libraries of the corresponding tumor. | | |
| **Obtained by random selection of clones from the CMS5a cDNA expression library. | | |
| ***Detected by antibody from patients with idiopathic thrombocytopenic purpura in SEREX analysis of cDNA expression libraries of the human megakaryoblastic leukemia cell line. | | |

### [Example 2] Enhancement of tumor-specific CD8⁺ T cell production by SEREX-defined immunogenic wild-type cellular molecules

cDNA encoding the whole protein of mERK2 was digested with *EcoR* I and *BamH* I, and then cloned into the *EcoR* I and *BamH* I site of pCAGGS-New (H. Niwa et al., Gene 106: 193-200 (1991)). The mERK2 plasmid was maintained in DH5α (TOYOBO, Osaka, Japan) and purified using QIAGEN Endfree Mega kit (QIAGEN, Hilden, Germany). 1µg of any of following (1) to (4) were mixed with 0.5mg of 1µm gold particles (BioRad, Hercules, CA) : (1) said mERK2 plasmid alone; (2) a mixture of said mERK2 plasmid and plasmid encoding either murine SEREX-defined molecules selected from the group of DNA J-like 2, DNA ligase 1, galectin-8 and poly (A) , or human SEREX-defined molecules selected from the group of hexamethylene-bis-acetamide (HMBA)-inducible, retinoic acid-responsive protein, delta antigen interacting protein A (DIPA) and FLJ20644fis; (3) a mixture of said mERK2 plasmid and plasmid encoding a molecule that was negative in SEREX (sorting nexin, glucose-regulated protein or Cctz-1) ; and (4) a mixture of said mERK2 plasmid and plasmid encoding chicken ovalbumin. The mixture was added to a centrifugate tube containing appropriate amount of 0.05 M spermidine (Nacalai Tesque, Kyoto, Japan) to adhere the plasmid DNAs to the gold particles (see, C. Condon et al., Nat. Med. 2: 1122-1128 (1997); A. Porgador et al., J. Exp. Med. 188: 1075-1082 (1998); D. Klinman et al., J. Immunol. 160: 2388-2392 (1998); C.A.T. Torres et al., J. Immunol. 158: 4529-4532 (1997); A. Iwasaki et al., J. Immunol. 159: 11-14 (1997)). The immunization of seven to nine week old BALB/c mice was performed by abdominal delivery of the plasmid-coated gold particles using Helios Gene Gun System (BioRad, Hercules, CA) at a helium discharge pressure of 350-400 psi. Mice received a booster injection 2 weeks after the initial immunization. A week later, CD8⁺ T cells were purified from the spleen of the mice.

Peptide 9m is a 9-mer peptide (amino acid sequence "QYIHSANVL") identified as an antigenic peptide specific for CMS5, a 3-methylchloranthrene-induced murine sarcoma, and was indicated as a tumor rejection antigen of CMS5 sarcoma (see, H. Ikeda et al. , Proc. Natl. Acad. Sci. USA 94: 6375-6379 (1997)). The. production of the peptide 9m was ordered to TaKaRa (Otsu, Japan). Furthermore, to confirm the specificity of the enzyme-linked immunospot assay, tumor rejection antigen against CMS17HER2, or HER2 p63-71(T) peptide was used as a control. HER2 p63-71(T) peptide is a 9-mer peptide identified as the target of CD8⁺ K^{d} restricted cytotoxic T cell (amino acid sequence "TYLPTNASL"; see Y. Nagata et al., J. Immunol. 159: 1336-1343 (1997); X. Gu et al., Cancer Res. 58: 3385-3390 (1998); Y. Ikuta et al., Int. J. Cancer 87: 553-558 (2000); T. Okugawa et al., Eur. J. Immunol. 30: 3338-3346 (2000); similar to 9m, ordered for production to TaKaRa)(hereinafter referred to as p63).

The 9m peptide was applied to P1.HTR, a mastosycoma cell line of DBA/2 origin (A. Van Pel et al., Somatic Cell. Genet. 11: 467-475 (1985)).

Cells secreting IFNγ among the above-described CD8⁺ T cell obtained from mice spleen were detected by ELISPOT assay using the P1.HTR cell stimulated with 9m peptide or p63-71(T). Thus, the existence of CD8⁺ cells specific to 9m peptide was assessed quantitatively.

By modifying the method of Power et al. (Power et al. , J. Immunol. Methods 227: 99-107 (1999)), ELISPOT assay was performed as follows. First, rat anti-murine INFα antibody (clone R 4-6A2; Pharmingen, San Diego, CA) was incubated at 4°C overnight in a nitrocellulose-coated 96-well microtiter plate (Millipore, Bedford, MA). After washing, the antibody was blocked at 37 °C for 1 to 2 hours. 1x10⁶ CD8⁺ T cells or 1x10⁶ P1.HTR were added to each well, incubated at 37°C for 24 hours and the wells were washed thoroughly. Then, biotinylated anti-murine IFNγ antibody (clone XMG1.2; Pharmingen, SanDiego, CA) was added and further incubated at 4°C overnight. After washing the wells, alkaline phosphatase-bound streptoavidine (MABTECH, Nacka, Sweden) was added and incubated at room temperature for 1.5 hours. Spots were generated by the addition of alkaline phosphatase-bound substrate kit (BioRad, Hercules, CA). The plate was thoroughly washed, dried and the number of formed spots counted using a dissecting microscope and Axloplan2 imaging system (Carl Zeiss vision, Hailbergmoss, Germany).

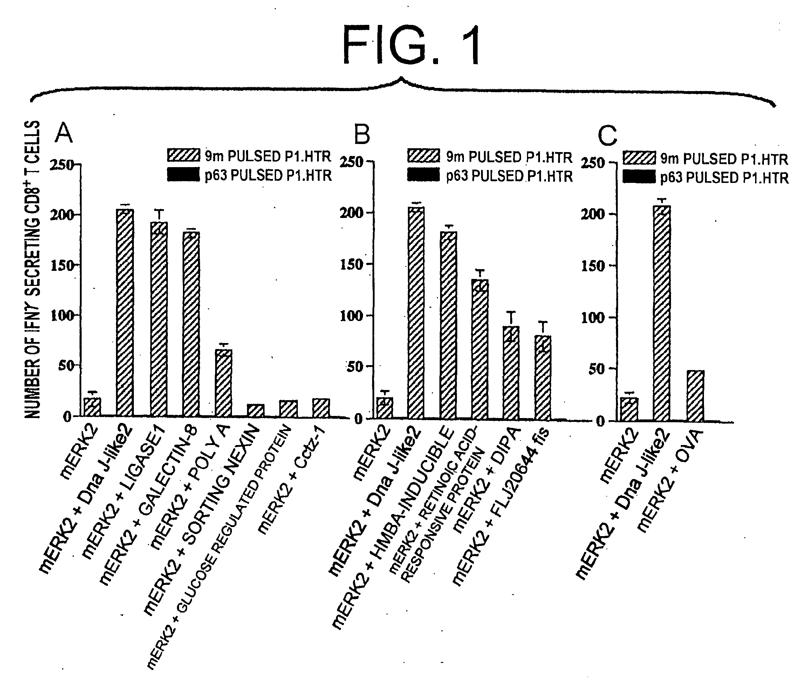

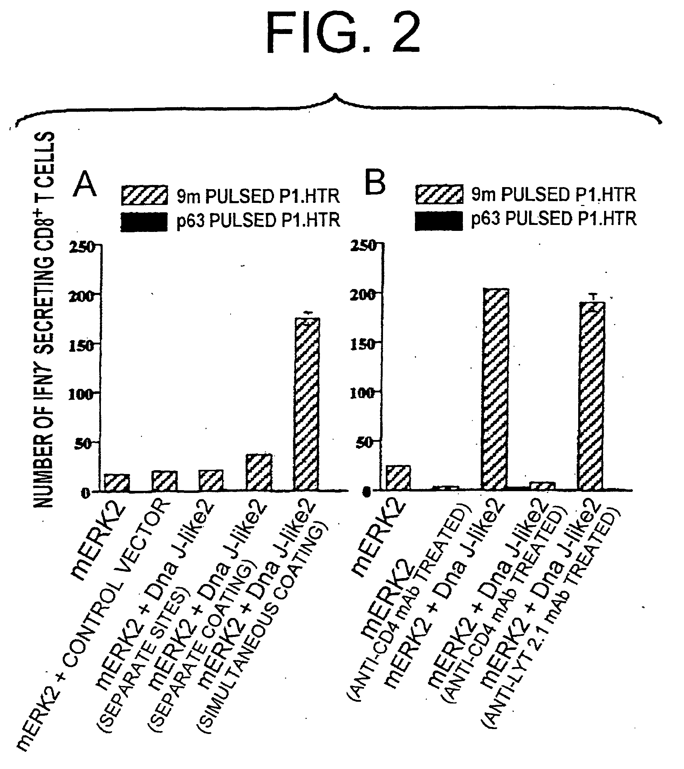

As a result, mice immunized with mERK2 cDNA alone generated low levels of 9m peptide-specific CD8⁺ T cells (Fig. 1). On the other hand, mice immunized with mERK2 and SEREX-defined antigens showed a striking increase in the number of 9m peptide-specific CD8⁺ T cells (Fig. 1a). The number of positive spots in these mice was three to 10 times higher than in mice immunized with mERK2 cDNA alone. In contrast, immunization with mERK2 plasmid mixed with a plasmid encoding a molecule that was not detected in the repeated SEREX analyses (e.g., sorting nexin, glucose-regulated protein or Cctz-1) did not show any increase of 9m peptide specific CD8⁺ T cells (Fig. 1a). Co-immunization with the control vector did not increase the number of 9m peptide specific CD8⁺ T cells (Fig. 2a). There were remarkably few number of spots detected in the p63-applied target cells. Thus, the specificity of the present ELISPOT assay was confirmed.

The cDNA encoding human SEREX-defined immunogenic heterologous molecules also greatly increased 9m peptide specific CD8⁺ T cell responses when presented together with mERK2 (Fig. 1b). In contrast, co-immunization with chicken ovalbumin resulted only . in a marginal increase in specific CD8⁺ T cell reactivity (Fig. 1c).

In Fig. 1, each bar represents the number of IFNγ-secreting CD8⁺ T cells per 10⁵ CD8⁺ T cells (Fig. 1a). Target cells were 9m-pulsed P1.HTR (hatched bars) or p63-pulsed P1.HTR (solid bars) as a control. Data are indicated as mean ±SEM of three experiments.

### [Example 3] Confirmation on the requirement of the co-presentation of SEREX-defined molecules and CD8⁺ T cell epitopes for enhancing CD8⁺ T cell response

To determine whether the CD8⁺ T cell epitopes and SEREX-defined molecules need to be co-presented on the same gold particle, mice were immunized by either of the following methods:
(1) immunization using a mixture of gold particles coated with mERK2 plasmid alone and gold particles coated with Dna J-like 2 plasmid alone (individually coated) ; or
(2) injection of particles coated with mERK2 plasmid alone and particles coated with Dna J-like 2 plasmid alone on opposite sides of the abdomen (different sites).
The production 'of gold particles coated with plasmids and immunization were performed similar to the method as described in Example 2. Moreover, the same target cells were used. Then, the number of 9m peptide-specific CD8⁺ T cells were counted.

Increase in the number of 9m peptide-specific CD8⁺ T cells was observed only in animals immunized with particles that were coated with both the plasmid encoding the antigen recognized by CD4⁺ T cells and plasmid encoding the antigen recognized by CD8⁺ T cells. No increase in CD8⁺ T cells specific for 9m peptides was observed in either of the above-described (1) or (2) (Fig. 2a). Thus, CD4⁺ T cells were confirmed to recognize SEREX-defined antigenic peptides presented by cells that also present a 9m T cell epitope derived from mERK2. Data are represented by mean ±SEM of three experiments.

### [Example 4] CD4⁺ T cell dependence of enhanced CD8⁺ T cell response

The present inventor examined whether CD4⁺ T cells were involved in promoting induction of 9m peptide-specific CD8⁺ T cells by SEREX-defined antigens. BALB/c mice were treated with anti-CD4 antibody (GK1.5) or anti-Lyt2.1 antibody as a control. Then, as described in Example 2, mice treated with either of the aforementioned antibodies were immunized with mERK2 plasmid and Dna J-like 2 plasmid. The same target cell as in Example 2 was used for this experiment.

When mice were treated with anti-CD4 antibody (GK1.5) before the immunization with the mixture of plasmid encoding mERK2 and plasmid encoding the SEREX-defined antigen Dna J-like 2, there was no increase in 9m peptide-specific CD8⁺ T cells. However, the control antibody (Lyt-2.1) did not inhibit the increase of 9m peptide-specific CD8⁺ T cells (Fig. 2b). Thus, the increase of CD8⁺ T cells due to immunization with mERK2 plasmid and Dna J-like 2 plasmid was shown to depend on CD4⁺ T cells. Data are represented by mean ±SEM of three experiments.

### [Example 5] Relation of immunogenic helper wild-type cellular molecules on primary and secondary tumor-specific responses of CD8⁺ T cells

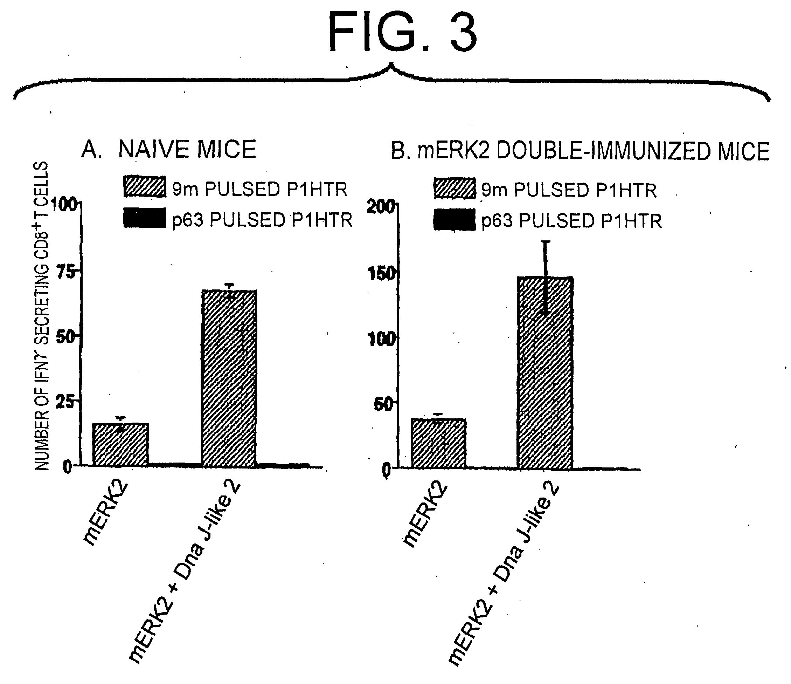

Naive mice (Fig. 3a) and mice that were twice preimmunized with plasmid encoding mERK2 (Fig. 3b) were used as models for primary and secondary responses, respectively. According to the method described in Example 2, mice were immunized with plasmid coding mERK2 or plasmids encoding mERK2 and Dna J-like 2. 14 days after the immunization, mice were similarly analyzed for 9m-specific CD8⁺ T cells. The results are shown in Fig. 3. Data are represented by mean ±SEM of three experiments. In both the models, the mice immunized with the plasmid encoding mERK2 alone, the number of 9m-specific CD8⁺ T cells significantly increased in mice immunized with plasmids encoding mERK2 and Dna J-like 2.

### [Example 6] Enhancement of HER2-specific CD8⁺ T cell induction by immunogenic wild-type molecules

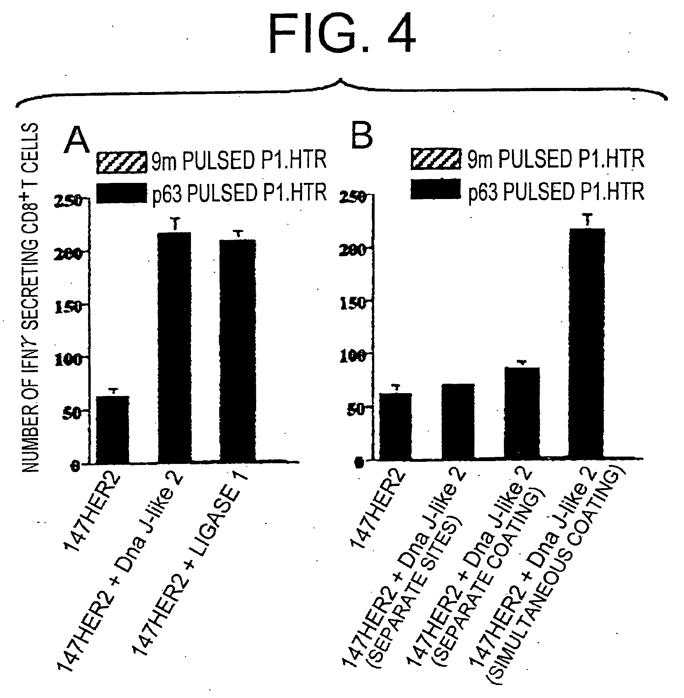

A cDNA plasmid encoding 147 N-terminal amino acid residues of human HER2 (hereinafter referred to as 147HER2) was prepared. According to the method described in Example 2, 147HER2 was immunized together with a plasmid encoding Dna J-like 2 or ligase 1, or without plasmid coding any of the SEREX-defined molecules. The same target cell as in Example 2 was used and the number of IFNγ-secreting CD8⁺ T cells was similarly counted. Increase in HER2 p63-specific CD8⁺ T cells was observed by the co-immunization with either of the SEREX-defined molecules (Fig. 4a). Data are represented by mean ± SEM of three experiments.

Similar to mERK2 in Example 3, the increase of HER p63-specific CD8⁺ T cells was examined in: (1) mice immunized with gold particles that were coated with both 147HER2 plasmid and SEREX-defined molecule plasmid; (2) mice immunized with individually coated gold particles; and (3) mice wherein the individually coated gold particles were respectively injected at distant parts of the abdomen (Fig. 4b). Data are represented by mean ± SEM of three experiments. The increase in the number of p63-specific CD8⁺ T cells was observed only for mice immunized with particles double-coated with plasmids of the two different molecules.

Considering this result with the result observed for mERK2 target tumor-specific immunity, it is strongly indicated that the immune response induced by immunogenic wild-type cellular molecules have the function to promote generation of CD8⁺ T cells in anti-tumor immune response against various tumors.

### [Example 7] In vivo tumor rejection

After in vivo injection, CMS5m tumor cell establishes metastases in the lungs, leading to death of animals within 5 to 6 weeks. Thus, BALB/c mice were challenged with 1x10⁶ CMS5m tumor cells in a total volume of 0.1 ml injected through the lateral caudal vein and used as models for pulmonary metastasis. Biweekly immunization with (1) plasmid encoding mERK2, (2) a mixture of mERK2 plasmid and control vector, or (3) a mixture of mERK2 plasmid and Dna J-like 2 plasmid, following the method described in Example 2 was commenced 7 or 14 days before, on the day of tumor challenge or 5 days after tumor challenge. After 28 days, mice were killed, and the number of pulmonary nodules was counted under a dissecting microscope. Each group included 5 animals, and the results are represented as mean ±SEM of the 5 animals.

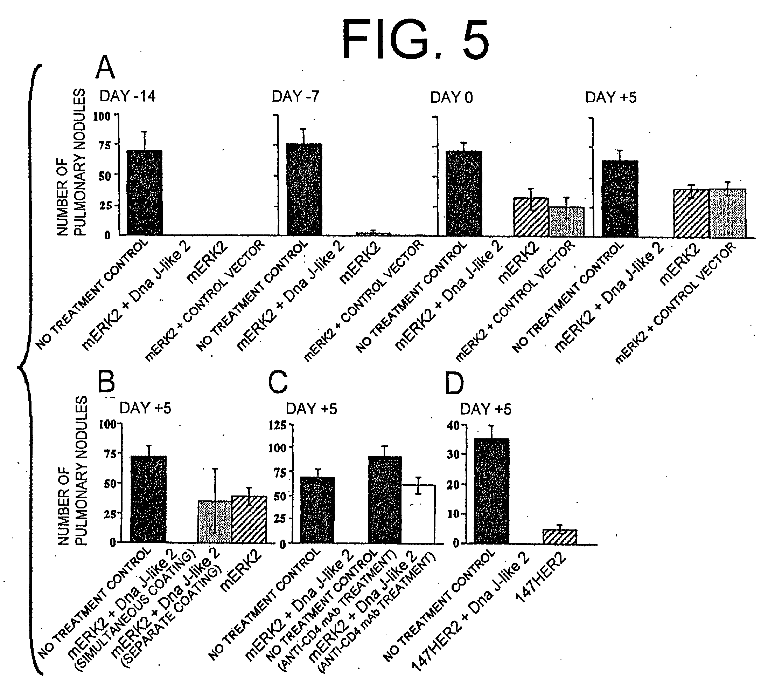

Immunization of mice with plasmid encoding mERK2 initiated 14 days before tumor challenge led to complete prevention of pulmonary metastasis. This protective effect was lost when immunization was initiated 7 days before tumor challenge or after tumor challenge (Fig. 5a). In contrast, immunization using a combination of plasmids of mERK2 and Dna J-like 2 plasmids showed complete prevention of metastases even when initiated as late as 5 days after tumor challenge (Fig. 5a). Absence of metastasis was confirmed by histopathological examination. The numbers in Fig. 5a are expressed as mean ± SEM of the five mice in each group.

Next, similarl to the above method except for the use of gold particles coated together with mERK2 plasmid and Dna J-like 2 plasmid, and mixtures of separately coated gold particles, their therapeutic effects were determined by immunization of mice after 5 days from tumor challenge. Pulmonary metastasis was not completely suppressed when the separately coated gold particles were used. It was demonstrated that co-presentation of mERK2 and Dna J-like 2 on the same gold particle is required (Fig. 5b). The numbers in Fig. 5b are expressed as mean ± SEM of the five mice in each group.

Furthermore, according to the method described in Example 4, mice were treated with anti-CD4 mAb (GK1.5) and then, after 5 days of tumor challenge, were immunized with gold particles co-coated with mERK2 and Dna J-like 2 to examine its therapeutic effect. As a result, the therapeutic effect of mERK2 and Dna J-like 2 was lost due to the treatment by anti-CD4 mAb (GK1.5). This shows that the therapeutic effect of mERK2 and Dna J-like 2 is CD4⁺ T cell-dependent (Fig. 5c). The numbers in Fig. 5c are expressed as mean ± SEM of the five mice in each group.

In addition, the therapeutic effects of plasmid encoding 147HER2, and a mixture of 147HER2 plasmid and Dna J-like 2 plasmid were examined by immunization of mice after 5 days of tumor challenge. The procedure was performed as described above except that the animals were sacrificed after 20 days of tumor challenge. As a result, similar to CMS5mHE, increased therapeutic effect was observed (Fig. 5d). The numbers in Fig. 5d are expressed as mean ± SEM of the five mice in each group.

As stated above, pulmonary metastasis of tumors that could not be completely suppressed by immunization with T cell epitopes alone was completely inhibited by conducting immunization using plasmid encoding an antigen recognized by a CD4⁺ helper T cell and plasmid encoding an antigen recognized by a CD8⁺ cytotoxic T cell coated on same particle.

### Industrial Applicability

As described above, the present invention revealed the critical role of CD4⁺ T cells in tumor-specific immune response by CD8⁺ T cells against immunogenic wild-type cellular molecules. A phenomenon similar to the CD8⁺ T cell immune response against tumor antigen heightened by co-recognition of SEREX-defined antigen in mice appears to exist in human. The heightened immune response results from the simultaneous incorporation by APC of complex antigenic mixture of destructed tumor cells. The resistance to tumor challenge increases in mice co-immunized with SEREX antigen and tumor antigen. This approach is an attractive strategy for cancer immunotherapy in humans and one that will be facilitated by the extensive base of information about SEREX-defined humor tumor antigens.
The vaccine of the present invention for eliciting tumor-specific immunity provides an effective immunotherapy method against tumors. The vaccine of the present invention shows both preventive and therapeutic effects. Thus, it is expected to provide a radical treatment for early stage cancers, therapy for suppressing postoperative recurrence or metastasis of cancers, and treatment for patients detected to have tumor but who cannot be operated and for whom radical and chemical treatments were found ineffective.

## Claims

1. A composition comprising an expression vector encoding (1) an antigen recognized by CD4⁺ helper T cells, and (2) an antigen recognized by CD8⁺ cytotoxic T cells.

2. The composition of claim 1, wherein the antigen recognized by CD4⁺ helper T cells is defined by the SEREX method.

3. The composition of claim 1 or 2, wherein the antigen recognized by CD8⁺ cytotoxic T cells is a tumor antigen.

4. The composition of claim 3, wherein the tumor antigen recognized by CD8⁺ cytotoxic T cells is HER2.

5. The composition of claims 3 or 4, wherein the tumor antigen recognized by CD8⁺ cytotoxic T cells is HER2 and Dna J-like 2.

6. The composition of any one of claims 1 to 5, wherein the polynucleotides encoding respective antigens are contained in different expression vectors.

7. The composition of claim 6, wherein both of the expression vectors are immobilized on the same carrier.

8. A vaccine comprising any one of the compositions described in claims 1 to 7.

9. The vaccine of claim 8, which is administered using a gene gun.

10. The vaccine of claim 8 or 9, which is used for the therapy and/or prevention of cancer.

11. A method for inducing tumor-specific immunity in a mammal, comprising the step of administering the composition of any one of claims 1 to 7 to the mammal.
